# EUROPEAN PATENT APPLICATION

(11) **EP 2 865 662 A1**
(43) Date of publication of application: **29.04.2015**
(21) Application number: 14189406.3
(22) Date of filing: 17.10.2014
(51) Int. Cl.: C07C 229/04, C07C 229/52, C07C 201/12, C07C 205/59, C07C 205/37, C07D 231/48

(54) **Process for the preparation of a thrombopoietin agonist**

(30) Priority: 25.10.2013 IT MI20131782
(71) Applicant: Dipharma Francis S.r.l., 20021 Baranzate (MI) (IT)
(72) Inventor: Allegrini, Pietro, 20021 BARANZATE (MI) (IT); Rossi, Roberto, 27100 PAVIA (IT); Attolino, Emanuele, 20021 BARANZATE (MI) (IT)
(74) Representative: Bertuccio, Silvia

(57) **Abstract**

The present invention relates to a process for the preparation of a thrombopoietin agonist, intermediates useful in its preparation, and a process for the preparation of said intermediates.

## Description

### FIELD OF INVENTION

The present invention relates to a process for the preparation of Eltrombopag, intermediates useful in its preparation, and a process for the preparation of said intermediates.

### PRIOR ART

3'-[(2Z)-[1-(3,4-dimethylphenyl)-1,5-dihydro-3-methyl-5-oxo-4H-pyrazol-4-idene]hydrazino]-2'-hydroxy-1,1'-biphenyl-3-carboxylic acid of formula (I), 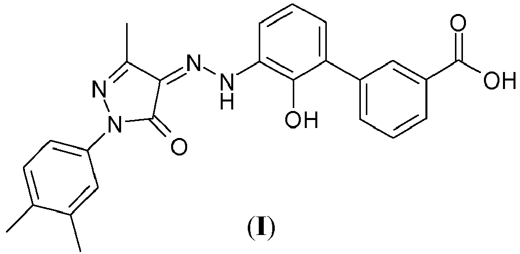 also known as Eltrombopag, is a thrombopoietin receptor agonist used in the treatment of patients suffering from immune thrombocytopenic purpura (ITP). ITP, which causes a rapid decline in platelets with no apparent justified cause, is a rare disease that can affect both children and adults.

Eltrombopag is marketed as the bisethanolamine salt in coated tablets containing 25 or 50 mg of Eltrombopag as free acid.

Eltrombopag is known from US 7,160,870, which describes its synthesis by a process that involves the diazo coupling reaction between the diazonium salt of formula **A** and the N-arylated derivative of 3-methyl-5-pyrazolone of formula **B.**

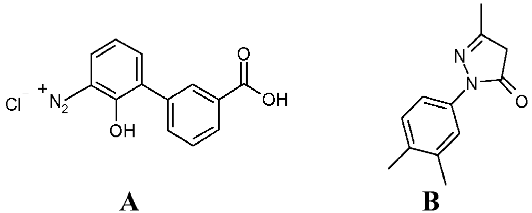

The diazonium salt of formula **A** is prepared in turn by treating the aniline of formula (II) with sodium nitrite in an acid environment. The pyrazolone of formula **B** is obtainable by methods well known to the skilled person, for example by condensation of 3,4-dimethylphenyl hydrazine with ethyl acetoacetate. The synthesis of the intermediate of formula **(II)** is clearly described in Example 3 of US 7,674,887, and is reported in the Scheme.

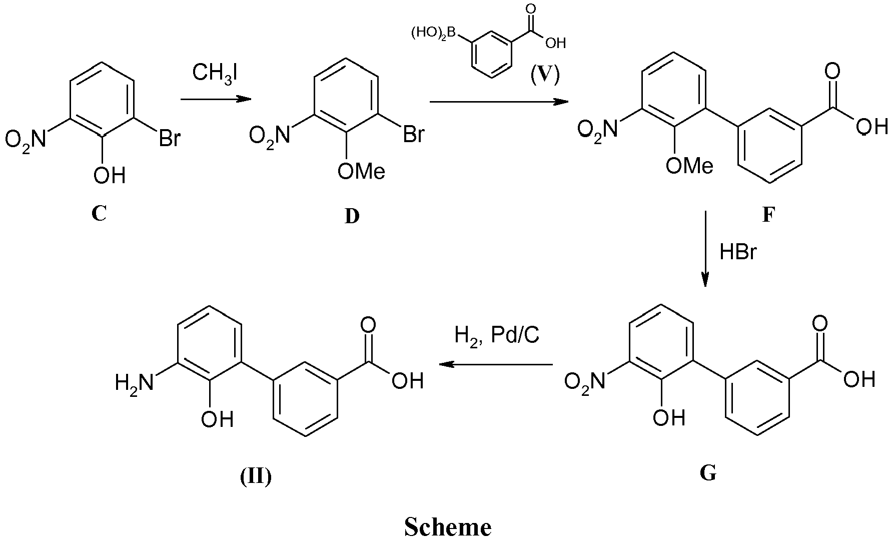

In accordance with said synthesis scheme, the phenolic hydroxyl of 2-bromo-6-nitrophenol of formula **C,** which is commercially available, is protected as methyl ether by reacting it with methyl iodide to obtain the protected intermediate of formula **D,** which can then undergo the cross-coupling reaction under Suzuki conditions with boronic acid **(V).** The protected biphenyl intermediate **F** is thus obtained. The deprotection of said intermediate of formula **F** with 48% aqueous HBr in acetic acid at reflux temperature produces the intermediate of formula **G,** which is converted by subsequent catalytic hydrogenation to the desired compound of formula **(II).**

If the synthesis process reported above is analysed in detail, it will be noted that it is somewhat laborious, because it consists of four synthesis steps. Moreover, the cross-coupling reaction of the compound of formula **D** with the expensive boronic acid of formula **(V)** gives a rather low yield (47%). Among other things, said process involves the use of methyl iodide, a known carcinogen. Last but not least, the inventors of the present invention have found that the deprotection reaction of the phenolic hydroxyl in a compound of formula **F** under the conditions reported above is carried out under particularly drastic conditions (i.e. in 48% HBr in acetic acid at reflux temperature), and leads to the formation of various impurities in the compound of formula **G.**

There is consequently a need for an alternative, improved preparation method, which is simpler and more advantageous, to prepare the compound of formula **(II)** and consequently Eltrombopag of formula **(I).** Said new method should in particular comprise a smaller number of synthesis steps, and the use of intermediates that can be synthesised and purified more easily on an industrial scale. Said process should also be cheap, safe for humans and the environment, and use mild reaction conditions to obtain Eltrombopag of formula **(I)** with high yields and efficiency.

### SUMMARY OF THE INVENTION.

The present invention relates to a process for the preparation of an intermediate of formula **(II),** or a salt thereof, 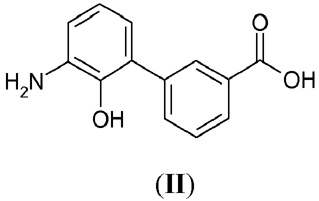 comprising hydrogenation of a compound of formula **(III),** or a salt thereof, 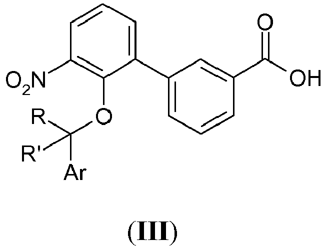 wherein Ar, R and R' are as defined below, and optionally the conversion of a compound of formula **(II)** thus obtained to the salt thereof or vice versa; the present invention also relates to a process for the preparation of intermediates of formula **(III),** and of formula **(IV),** or a salt thereof, 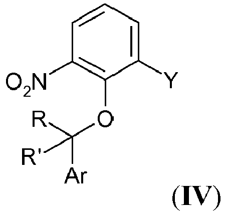 wherein Ar, R, R' and Y are as defined below, which are useful in the preparation of the compound of formula (II), or a salt thereof, and their use in a process for the preparation of Eltrombopag of formula (I), or a salt thereof,

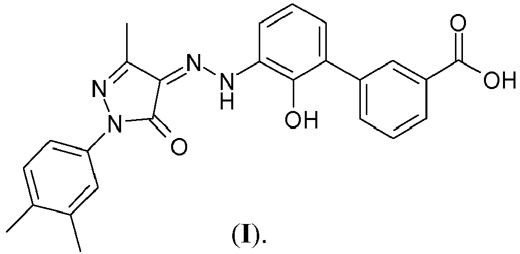

### BRIEF DESCRIPTION OF FIGURES AND ANALYTIC METHODS

The crystalline form of 2'-benzyloxy-3'-nitrobiphenyl-3-carboxylic acid of formula (III) and the crystalline form of benzyl 2-bromo-6-nitrophenyl ether of formula **(IV)** to which this invention also relates have been characterised by XRPD analysis. The X-ray powder diffraction spectra (XRPD) of said crystalline forms were collected with a Bruker D8 Advance automatic powder diffractometer, CuKa radiation (λ= 1.54 Å), scanning with a 2θ angle range of 3-40°, step size of 0.02°, for a time of 0.5 sec. The detector used was a LynxEye PSD.
Figure 1: XRPD of the crystalline form of 2'-benzyloxy-3'-nitrobiphenyl-3-carboxylic acid of formula **(III);** wherein the most intense peaks expressed in 2θ are found at about 6.44; 8.42; 12.87; 15.57; 15.91; 18.35; 19.35; 20.25; 20.88; and 22.46 ± 0.2°.
Figure 2: XRPD of the crystalline form of benzyl 2-bromo-6-nitrophenyl ether of formula (IV); wherein the most intense peaks, expressed in 2θ, are found at about 12.09; 13.21; 13.49; 15.42; 16.55; 17.81; 19.84; 21.28; 22.64; and 22.85 ± 0.2°.

### DETAILED DESCRIPTION OF THE INVENTION

The subject of the present invention is a process for the preparation of a compound of formula **(II),** or a salt thereof, 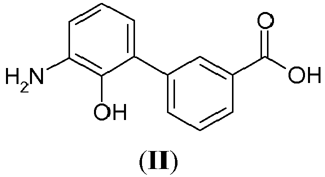 comprising hydrogenation of a compound of formula (III), or a salt thereof, 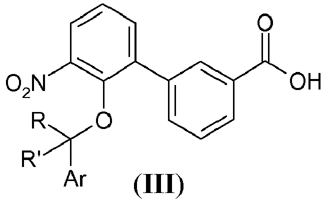 wherein each of R and R', which are equal or different, is independently hydrogen, a C₁-C₆ alkyl group, or an aryl group; and Ar is an aryl group; and optionally, the conversion of a compound of formula **(II)** thus obtained to a salt thereof or vice versa.

A C₁-C₆ alkyl group, which can be straight or branched, is typically a C₁-C₄ alkyl group such as methyl, ethyl, propyl, isopropyl or butyl, isobutyl, tert-butyl, which can be substituted by one or more substituents, preferably by one to three substituents, for example a halogen group such as chlorine or fluorine.

An aryl group can be, for example, a phenyl group or a naphthyl group. Said aryl group can be optionally substituted by one to three substituents selected independently from a straight or branched C₁-C₄ alkyl group, which in turn is optionally substituted by one to three atoms of halogen, typically fluorine; a hydroxy group; a C₁-C₄ alkoxy group, such as methoxy; a halogen atom, such as bromine or chlorine; a cyano group and a nitro group.

The hydrogenation of the compound of formula **(III)** or a salt thereof is typically catalytic, and can optionally be carried out under hydrogen transfer conditions.

Catalytic hydrogenation is typically carried out in the presence of hydrogen (H₂) and a homogeneous or heterogeneous metal catalyst, for example based on palladium (Pd), platinum (Pt), nickel (Ni), rhodium (Rh) or ruthenium (Ru). Said hydrogenation is preferably carried out in the presence of a metal catalyst based on Pd. When the metal catalyst is heterogeneous, it is preferably deposited on an inert support such as charcoal, barium hydroxide, alumina or calcium carbonate, preferably charcoal.

In a particularly preferred aspect of the present invention, the metal catalyst is a heterogeneous metal catalyst, more preferably palladium supported on carbon (Pd/C).

The concentration of metal on the inert support can range between about 1% and 30%, preferably between about 5% and 20%.

The hydrogen pressure used can range between about 1 bar and 10 bars; the reaction is preferably carried out between about 2 bars and 6 bars, preferably at about 4 bars.

The molar quantity of catalyst used, as a proportion of the compound of formula **(II)** or a salt thereof, typically ranges between about 0.1 and 10%, preferably between about 0.5 and 5%.

The hydrogenation reaction can be carried out in the presence of an organic solvent selected, for example, from a polar aprotic solvent, typically dimethylformamide, dimethylacetamide, acetonitrile or dimethyl sulphoxide; a cyclic or acyclic ether, typically tetrahydrofuran, dioxane or methyl-tertbutyl ether; a chlorinated solvent, typically dichloromethane; an apolar aprotic solvent, typically toluene or hexane; a polar protic solvent, such as a straight or branched C₁-C₆ alkanol, in particular methanol, ethanol, isopropanol, n-butanol or tert-butanol; water; an ester, such as ethyl acetate, isopropyl acetate or butyl acetate; a straight or branched C₃-C₇ ketone, such as acetone, methylethyl ketone or methyl isobutyl ketone; a carboxylic acid, such as acetic acid or propionic acid; or a mixture of two or more, preferably two or three, of said solvents.

Alternatively, the reaction can be carried out in a solution of a mineral acid, such as hydrochloric acid or sulphuric acid, or a mixture thereof with one or more, preferably, two or three, of the organic solvents cited above. The reaction can preferably be carried out in a straight or branched C₁-C₆ alkanol, as defined above, or in a mixture containing at least two of the C₁-C₆ alkanols as defined above, or in a mixture of at least one C₁-C₆ alkanol as defined above with water, or in an acetonitrile/water mixture; the hydrogenation reaction is preferably carried out in methanol.

Said hydrogenation reaction of a compound of formula **(III),** or a salt thereof, as defined above, can be carried out at a temperature of between about 0°C and the reflux temperature of the solvent, preferably between about 10°C and 60°C, and more preferably at about 50°C.

The catalytic hydrogenation reaction of a compound of formula **(III),** or a salt thereof, can optionally be carried out under hydrogen transfer conditions using a homogeneous or heterogeneous metal catalyst, for example as defined above, and a hydrogen donor.

A hydrogen donor in accordance with the present invention is typically selected from the group comprising cyclohexene; cyclohexadiene; methylcyclohexene; limonene; dipentene; mentene; hydrazine; phosphinic acid or derivatives thereof, such as sodium hypophosphite: indoline; ascorbic acid; formic acid or its sodium or ammonium salts; and a secondary alcohol, such as isopropanol. The hydrogen donor is preferably ammonium formate.

The molar quantity of catalyst used in the hydrogen transfer conditions is typically between about 0.1 and 10%, preferably between about 0.5 and 5%, of the compound of formula **(II),** or a salt thereof.

The molar ratio between the hydrogen donor and the compound of formula **(III),** or a salt thereof, can range between about 1.5 and 50, preferably between about 1.5 and 10.

The catalytic hydrogenation reaction in hydrogen transfer can be carried out in the presence of an organic solvent chosen, for example, from the group mentioned above.

The hydrogenation reaction of a compound of formula **(III),** or a salt thereof, in accordance with the methods reported above, leads to the formation of the compound of formula **(II),** or a salt thereof, by simultaneous reduction of the nitro group to an amino group and removal of the alkyl (CRR'Ar) grouping from the phenolic hydroxyl of the compound of formula **(III).**

The compound of formula **(II),** or a salt thereof, obtained by the process according to the present invention, after crystallisation presents a chemical purity equal to or greater than 98%, as evaluated by HPLC analysis.

A compound of formula **(II),** or a salt thereof, obtained by the process according to the present invention, can be efficiently used in the synthesis of Eltrombopag, for example as described in US 7,674,887.

A further subject of the present invention is therefore the conversion of a compound of formula **(II),** or a salt thereof, obtained by the process according to the present invention, to Eltrombopag of formula **(I),** or a salt thereof.

A compound of formula **(III),** or a salt thereof, preferably in crystalline form, is novel, and constitutes a further subject of the present invention. Said compound, in particular in crystalline form, produces Eltrombopag of formula **(I),** or a salt thereof, with a high yield and purity.

According to a preferred aspect of the invention, the compound of formula **(III)** is 2'-benzyloxy-3'-nitrobiphenyl-3-carboxylic acid, namely a compound of formula **(III)** wherein R and R' are both H and Ar is phenyl, preferably in crystalline form.

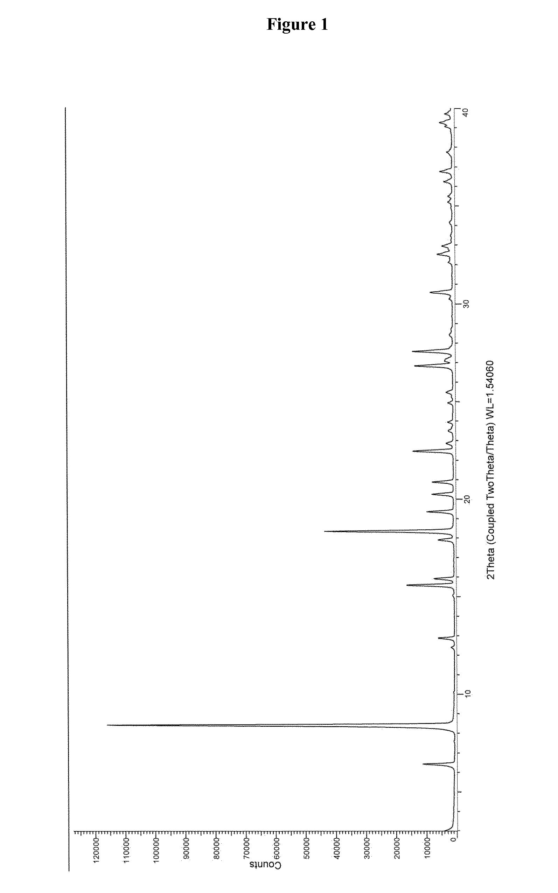

The present invention relates in particular to 2'-benzyloxy-3'-nitrobiphenyl-3-carboxylic acid of formula **(III)** in crystalline form having an XRPD spectrum as shown in Figure 1, wherein the most intense peaks expressed in 2θ are found at about 6.44; 8.42; 12.87; 15.57; 15.91; 18.35; 19.35; 20.25; 20.88; and 22.46 ± 0.2°.

A further subject of the present invention is a process for the preparation of a compound of formula **(III),** or a salt thereof, comprising the reaction between a compound of formula **(IV),** 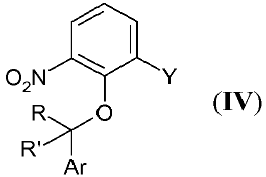 wherein R, R' and Ar are as defined above; and Y is a leaving group; with the boronic acid of formula **(V),** or a salt thereof, under Suzuki conditions.

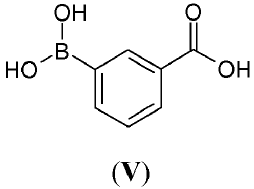

A leaving group Y according to the present invention is typically a halogen atom, preferably bromine or iodine, or an OSO₂Z group, wherein Z is, for example, methyl, ethyl, phenyl, benzyl, p-tolyl, p-bromophenyl, p-nitrophenyl, trifluoromethyl, nonafluorobutyl or N-imidazole, preferably methyl.

The Suzuki reaction between a compound of formula **(IV)** and a compound of formula **(V)** can be carried out by a method known to the skilled person; for example it can be carried out in the presence of a catalyst based on Pd(0), preformed or generated *in situ* from a Pd(II) salt, in particular PdCl₂ or Pd(OAc)₂, and, if desired, in the presence of a binder, such as triphenylphosphine, and an organic or inorganic base, in particular selected from the group comprising a secondary amine, a tertiary amine, a hydroxide or an alkali metal carbonate. Said reaction can optionally be carried out in the presence of a solvent, such as a dipolar aprotic solvent, typically dimethylformamide, dimethylacetamide, acetonitrile or dimethyl sulphoxide; an ether solvent, typically tetrahydrofuran or dioxane; an alcohol-based solvent, typically a C₁-C₄ alkanol, such as isopropanol; water, or a mixture of two or more, preferably two or three, of the solvents listed above.

According to the present invention, the Suzuki reaction is preferably carried out in a mixture of tetrahydrofuran and water, using potassium carbonate as base.

Under these reaction conditions the compound of formula **(III)** or a salt thereof, in particular in crystalline form, is obtained with high yields and chemical purity.

In particular, starting from the compound of formula **(IV),** wherein the leaving group Y is bromine, R and R' are both H and Ar is phenyl, the compound of formula **(III)** is obtained after crystallisation with a yield of 80% and chemical purity equal to or greater than 98% evaluated by HPLC, and the compound of formula **(II)** is obtained with a similarly high yield and purity.

However, if the same reaction conditions are used, but starting with the intermediate protected as methyl ether of formula **D** reported in the Scheme above, similar to the compound of formula **(IV),** the authors of the present invention have found that the compound of formula **(II)** is obtained with a very low yield, amounting to about 50%.

The process according to the present invention is therefore particularly advantageous, and the result obtained is surprising and wholly unexpected, especially as regards the process yields and the purity of the products obtained.

A compound of formula **(IV),** or a salt thereof, in particular in crystalline form, is novel, and constitutes a further subject of the present invention.

According to a preferred aspect of the invention, the compound of formula **(IV)** is benzyl 2-bromo-6-nitrophenyl ether, namely a compound of formula **(IV)** wherein R and R' are both H, Ar is phenyl and Y is bromine, preferably in crystalline form.

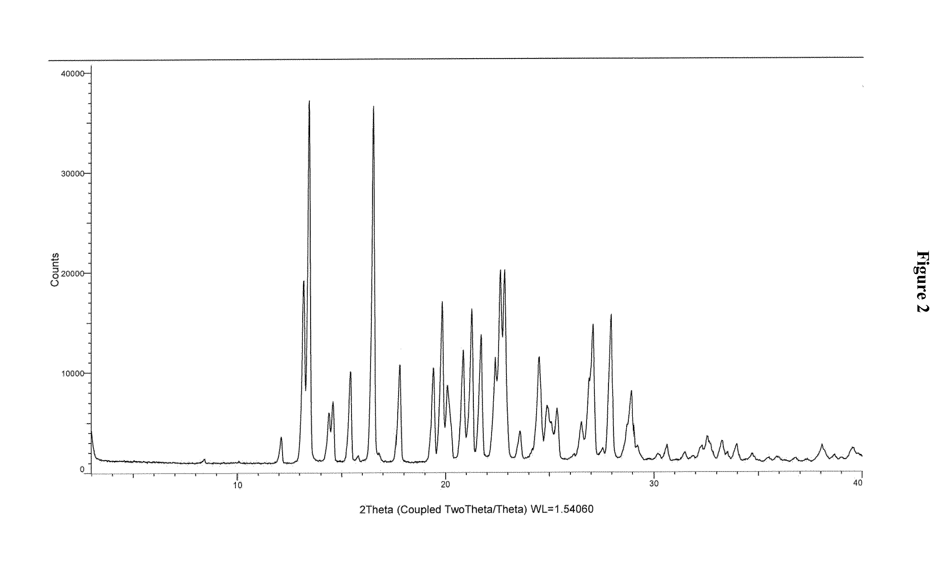

The present invention relates in particular to benzyl 2-bromo-6-nitrophenyl ether of formula **(IV)** in crystalline form having an XRPD spectrum as shown in Figure 2, wherein the most intense peaks, expressed in 2θ, are found at about 12.09; 13.21; 13.49; 15.42; 16.55; 17.81; 19.84; 21.28; 22.64; and 22.85 ± 0.2°.

It should be noted that the compounds of formula **(III)** and **(IV),** or a salt thereof, can be efficiently converted to the compound of formula **(II),** or a salt thereof, which can then be advantageously used in the synthesis of Eltrombopag of formula **(I),** or a salt thereof.

A further subject of the invention is therefore the use of a compound of formula **(III)** or **(IV),** or a salt thereof, as intermediate in a process for the preparation of Eltrombopag of formula **(I),** or a salt thereof.

A compound of formula **(V),** or a salt thereof, is commercially available, while a compound of formula **(IV)** can be prepared by etherification of 2-bromo-6-nitrophenol with a compound of formula **(VI)** 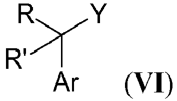 wherein Y, R, R' and Ar are as defined above.

The etherification reaction can be carried out under conditions well-known to the skilled person to protect the phenolic hydroxyl as ether, for example in the presence of an organic or inorganic base in a solvent.

A salt of a compound of formula **(I), (II), (III), (IV)** or **(V)** is preferably a pharmaceutically acceptable salt thereof, for example a salt known to the prior art, such as the sodium, potassium, ethanolamine or bisethanolamine salt.

A salt of a compound of formula **(I), (II), (III), (IV)** or **(V)** can be converted to the corresponding free compound by methods known to the prior art, such as release of salt under acid conditions, for example by adding hydrochloric acid.

A compound of formula **(I), (II), (III), (IV)** or **(V)** can be converted to a salt thereof by methods known to the prior art, for example by treating the compound in free form with a base.

The following examples further illustrate the invention.

### Example 1: Synthesis of benzyl 2-bromo-6-nitrophenyl ether of formula (IV)

2-bromo-6-nitrophenol (100 g, 0.459 mol), K₂CO₃ (74.2 g, 0.537 mol), KI (3.81 g, 23 mmol) and benzyl chloride (62.7 g, 0.495 mol) are suspended in acetone (600 ml) in a 2 L flask with mechanical stirrer, condenser and thermometer, purged under nitrogen atmosphere. The reaction mixture is heated at reflux temperature for 15 hours, and then cooled to about 25°C and filtered through a Celite bed. The panel is further washed with acetone, and the organic phases are combined and concentrated at low pressure. The residue thus obtained is crystallised from hexane (600 ml). The solid product is filtered and stove-dried at 40°C at low pressure. 126 g of benzyl 2-bromo-6-nitrophenyl ether **(IV)** is obtained, with a yield of 90%.

The compound of formula **(IV)** thus obtained presents an XRPD spectrum substantially as reported in Figure 2, wherein the most intense peaks expressed in 2θ are found at about 12.09; 13.21; 13.49; 15.42; 16.55; 17.81; 19.84; 21.28; 22.64; and 22.85 ± 0.2° in 20.

¹H-NMR (CDCl₃, 300 MHz), δ: 7.86-7.75 (m, 2H); 7.67-7.50 (m, 2H) 7.46-7.32 (m, 3H); 7.14 (t, *J*=8.2 Hz, 1H); 5.19 (s 2H).

### Example 2: Synthesis of 2'-benzyloxy-3'-nitrobiphenyl-3-carboxylic acid of formula (III)

Triphenylphosphine (5.62 g, 21.4 mmol) is dissolved in THF (50 ml) in a 100 ml flask with magnetic stirrer, purged under nitrogen atmosphere, and palladium acetate (1.6 g, 7.14 mmol) is added. The suspension is maintained under stirring for one hour at room temperature, and then added to a suspension of benzyl 2-bromo-6-nitrophenyl ether **(IV)** (110 g, 0,357 mol), obtained as in example 1, and K₂CO₃ (128 g, 0.93 mol) in THF (660 ml), prepared in an inertised 1 L flask with mechanical stirrer, thermometer and condenser. The reaction mixture is then heated to reflux temperature and treated with 3-carboxyphenylboronic acid (65.2 g, 0.393 mol), added solid in portions. The reaction mixture is maintained at the same temperature for 12 hours, and then cooled to about 25°C, diluted with water (400 ml) and treated with concentrated H₂SO₄ (92 g, 0.94 mol). The solvent is then distilled at low pressure, and the product is extracted in dichloromethane (3 x 300 ml). The combined organic phases are washed with water, dried on sodium sulphate, filtered and concentrated at low pressure. The solid residue thus obtained is suspended in water (500 ml), and the suspension is heated at reflux temperature until completely dissolved. The solution is then cooled to about 0°C, and the solid product obtained is filtered through a Büchner funnel. The solid is washed with water and stove-dried at 50°C. 100 g of 2'-benzyloxy-3'-nitrobiphenyl-3-carboxylic acid of formula **(III)** is obtained, with a yield of 80% and HPLC purity exceeding 98%.

The compound of formula **(III)** thus obtained presents an XRPD spectrum substantially as reported in Figure 1, wherein the most intense peaks, expressed in 2θ, are found at about 6.44; 8.42; 12.87; 15.57; 15.91; 18,35; 19.35; 20.25; 20.88; and 22.46 ± 0.2°.

¹H-NMR (DMSO-d_{6,} 300 MHz), δ: 8.12 (s, 1H); 8.05 (d, 1H); 7.92 (d, 1H); 7.82 (d, 1H), 7.76 (d 1H), 7.60 (t, 1H), 7.45 (t, 1H), 7.30-7.19 (m, 3H), 7.02-6.94 (m, 2H), 4.65 (s, 2H).

### Example 3: Synthesis of 3'-amino-2'-hydroxybiphenyl-3-carboxylic acid of formula (II)

The solution obtained by dissolving in methanol (400 ml) the 2'-benzyloxy-3'-nitrobiphenyl-3-carboxylic acid (97 g, 0.278 mol) obtained as in Example 2 is placed in a 1 L hydrogenator. 5% Pd/C (23.68 g, 13.9 mmol Pd, moisture about 50%) is added to the solution. The suspension is purged under nitrogen atmosphere, placed at a hydrogen pressure of about 4 bars and maintained under stirring at about 50°C for 8 hours. The suspension is then cooled to about 25°C and filtered through a Celite bed. The panel is washed with hot methanol, and the organic phases are combined and concentrated at low pressure. The solid residue thus obtained is suspended in hexane (400 ml), and the resulting suspension is heated at 55°C for 2 hours. The suspension is then cooled to about 25°C and filtered, and the solid is stove-dried at 50°C. 50 g of 3'-amino-2'-hydroxybiphenyl-3-carboxylic acid of formula (II) is obtained, with a yield of 80% and HPLC chemical purity exceeding 98%.

¹H-NMR (DMSO-d₆, 300 MHz), δ: 8.12 (s, 1H), 7.81 (d, 1H) 7.63 (d, 1H), 7.45 (t, 1H), 6.75-6.60 (m, 2H), 6.45 (d, 1H).

## Claims

1. A process for preparing a compound of formula (II), or a salt thereof, 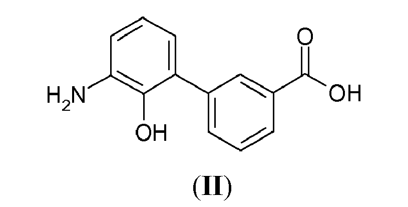 comprising the hydrogenation of a compound of formula (III), or a salt thereof, 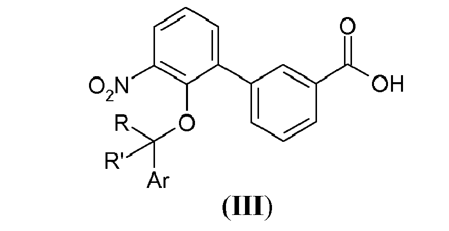 wherein each of R and R', being the same or different, is independently hydrogen, a C₁-C₆ alkyl group, or an aryl group; and Ar is an aryl group;
and optionally, the conversion of a so obtained compound of formula (II) to a salt thereof, or vice versa.

2. A process according to claim 1, wherein the hydrogenation is a catalytic hydrogenation carried out in the presence of hydrogen (H₂) and of a homogeneous or heterogeneous metal catalyst.

3. A process according to claim 2, wherein the metal catalyst is selected from a palladium (Pd), platinum (Pt), nickel (Ni), rhodium (Rh) and ruthenium (Ru) based catalyst, preferably a Pd based catalyst.

4. A process according claim 1, 2 or 3, wherein the hydrogenation is carried out in hydrogen transfer conditions.

5. A process according to claims 2, 3 or 4, wherein the metal catalyst is a heterogeneous metal catalyst, preferably palladium supported on carbon (Pd/C).

6. A process according to claims 2- 5, wherein the concentration of the metal on the inert support ranges between about 1% and 30%, preferably between about 5% and about 20%.

7. A process according to claims 2-6, wherein the molar amount of metal catalyst, referred to the compound of formula **(II)** or a salt thereof, is typically comprised between about 0.1 and 10%, preferably between about 0.5 and 5%.

8. Process according to claims 1-7, wherein the hydrogenation is carried out in the presence of an organic solvent, selected in the group comprising an aprotic polar solvent, typically dimethylformamide, dimethylacetamide, acetonitrile, dimethylsulphoxide; an acyclic or cyclic ether; a chlorinated solvent; an apolar aprotic solvent, typically toluene or hexane; a polar protic solvent, such as a straight or branched C₁-C₆ alkanol, preferably methanol; water; an ester; a straight or branched C₃-C₇ ketone; a carboxylic acid; or a mixture of two or more, preferably two or three, of said solvents; or, alternatively, the hydrogenation is carried out in a solution comprising a mineral acid or a mixture thereof with one or more of the organic solvents mentioned above.

9. A compound selected from:
- a compound of formula **(III),** or a salt thereof, in particular in crystalline form, 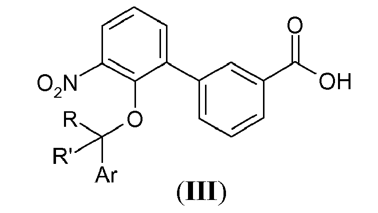 wherein each of R and R', being the same or different, is independently hydrogen, a C₁-C₆ alkyl group or an aryl group, and Ar is an aryl group; and
- a compound of formula **(IV),** or a salt thereof, in particular in crystalline form, 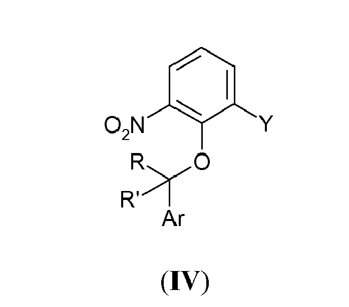 wherein R, R' and Ar are as defined above; and Y is a leaving group.

10. A compound of formula **(III),** or a salt thereof, as defined in claim 9, which is 2'-benzyloxy-3'-nitrobiphenyl-3-carboxylic acid, or a salt thereof.

11. A compound of formula **(III),** as defined in claim 10, having an XRPD spectrum as shown in Figure 1, wherein the most intense peaks expressed in 2θ are found at about 6.44; 8.42; 12.87; 15.57; 15.91; 18.35; 19.35; 20.25; 20.88; and 22.46 ± 0.2°.

12. A compound of formula **(IV),** or a salt thereof, as defined in claim 9, which is benzyl 2-bromo-6-nitrophenyl ether.

13. A compound of formula **(IV),** as defined in claim 12, having an XRPD spectrum as shown in Figure 2, wherein the most intense peaks, expressed in 2θ, are found at about 12.09; 13.21; 13.49; 15.42; 16.55; 17.81; 19.84; 21.28; 22.64; and 22.85 ± 0.2°.

14. A process according to claims 1-8, further comprising converting a compound of formula (II), or a salt thereof, to Eltrombopag of formula (I), or a salt thereof,

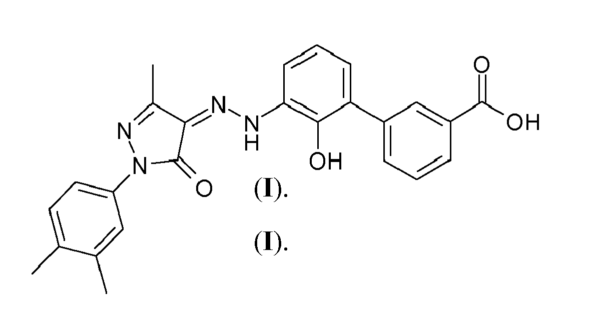

15. A process for preparing a compound of formula (III), or a salt thereof, as defined in claim 9, comprising reacting a compound of formula (IV), wherein 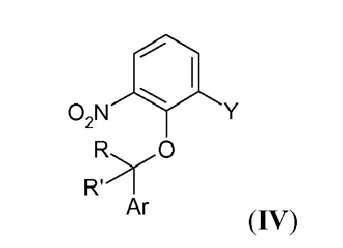 R, R', Ar and Y are as defined in claim 9, with the boronic acid of formula (V), or a salt thereof, 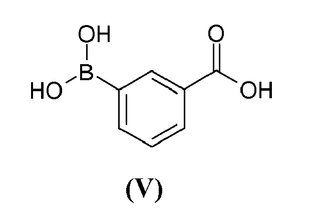 in Suzuki conditions.
